(19) **Europäisches Patentamt**
**European Patent Office**
**Office européen des brevets**

(11) **EP 2 500 061 B1**

(12) **EUROPÄISCHE PATENTSCHRIFT**

(45) Veröffentlichungstag und Bekanntmachung des Hinweises auf die Patenterteilung:
**25.11.2015 Patentblatt 2015/48**

(51) Int Cl.:
**A61N 5/10** (2006.01)

(21) Anmeldenummer: **12152429.2**

(22) Anmeldetag: **25.01.2012**

(54) **Verfahren und Vorrichtung zur Bestimmung eines Bestrahlungsplans**

Method and device for determining an irradiation plan

Procédé et dispositif destinés à la détermination d'un plan de traitement d'irradiation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.03.2011 DE 102011005739**

(43) Veröffentlichungstag der Anmeldung:
**19.09.2012 Patentblatt 2012/38**

(73) Patentinhaber: **Siemens Aktiengesellschaft 80333 München (DE)**

(72) Erfinder:
• **Fieres, Johannes 69115 Heidelberg (DE)**
• **Thilmann, Oliver 69126 Heidelberg (DE)**

(56) Entgegenhaltungen:
EP-A1- 1 818 078      US-A1- 2007 201 614
US-A1- 2010 104 068      US-B2- 7 268 358

**Beschreibung**

Die vorliegende Erfindung betrifft ein Verfahren zur Bestimmung eines Bestrahlungsplans für eine Bestrahlung eines Bestrahlungsobjekts und eine entsprechende Vorrichtung. Die vorliegende Erfindung betrifft insbesondere ein Verfahren zur Bestimmung eines Bestrahlungsplans zur Bestrahlung eines Patienten mit Mehrfachstrahlen im Rahmen einer Partikel- oder Teilchentherapie, einer so genannten Multi-Beam-Teilchentherapie.

US 7 268 358 B2 lehrt ein Verfahren gemäß dem Oberbegriff des Anspruchs 1.

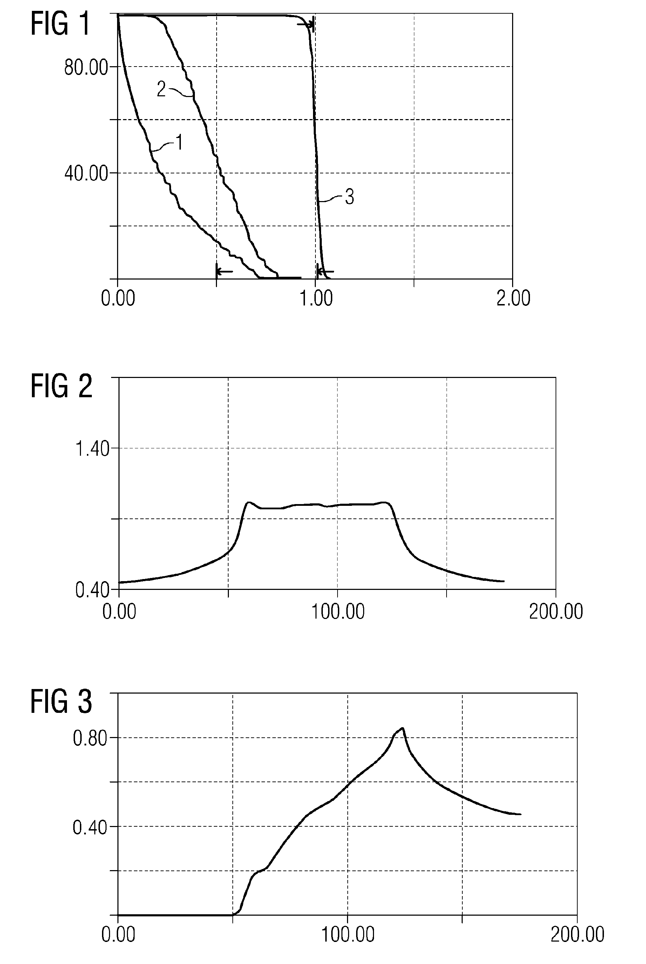

Bei einer Mehrfachstrahlteilchentherapie, welche auch Mehrfelderbestrahlung genannt wird, addiert sich von mehreren Strahlen mit unterschiedlichen Einfallswinkeln gelieferte Strahlung zu einer konformen Dosisverteilung über einem Zielvolumen. Die Teilchenfluenzprofile der Strahlen werden üblicherweise von einem automatischen Optimierungsverfahren bestimmt, welches auf der Grundlage von medizinischen Zielen arbeitet, die von einem menschlichen Benutzer definiert werden. Diese Ziele werden üblicherweise als Bedingungen in Bezug auf die resultierende Dosisverteilung ausgedrückt. Heutzutage gibt es zwei prinzipielle Ansätze, um die Dosisbedingungen zu spezifizieren: entweder spezifiziert der Benutzer die gewünschten Teildosen von jedem Strahl separat, zum Beispiel derart, dass sie homogen über dem Ziel sind (Einzelstrahloptimierung, Single-Beam-Optimization, SBO), oder der Benutzer spezifiziert die gewünschte Summendosis von allen Strahlen (intensitätsmodulierte Teilchentherapie, Intensity-Modulated Particle Therapy, IMPT) über dem Ziel. Bei der IMPT werden keine Bedingungen an die einzelnen Strahldosen gestellt und daher können die einzelnen Strahldosen sehr inhomogen sein. Beide Verfahren, SBO und IMPT, haben ihre Vorteile und Nachteile, welche unter Bezugnahme auf die Figuren 1-8 nachfolgend dargestellt werden.

Fig. 1 zeigt ein so genanntes Dosis-Volumen-Histogramm, bei welchem auf der x-Achse eine Strahlendosis in Gray (Gy) und auf der y-Achse ein Gewebevolumenanteil in Prozent aufgetragen wird. Die Graphen 1-3 geben jeweils für einen Gewebebereich an, wie viel Prozent des jeweiligen Gewebes (y-Achse) eine Strahlendosis gemäß dem Wert auf der x-Achse, oder höher, erhalten. Ein derartiges Dosis-Volumen-Histogramm (DVH) kann beispielsweise durch eine Simulation bei vorgegebenen Bestrahlungsparametern vor einer Bestrahlung bestimmt werden. In Fig. 1 zeigen die Graphen 1 und 2 beispielsweise ein Dosis-Volumen-Histogramm für sensitive Gewebebereiche oder Organe, d.h. für Gewebebereiche oder Organe, welche bei einer Bestrahlung im Bestrahlungsgang liegen, aber möglichst gering bestrahlt werden sollten, da sie nicht das Zielgebiet der Bestrahlung darstellen. Ziel ist es daher, dass diese Graphen im Dosis-Volumen-Histogramm möglichst weit links liegen, d.h., eine möglichst geringe Strahlung insgesamt und auch eine möglichst geringe maximale Strahlung erhalten. Graph 3 zeigt hingegen das Dosis-Volumen-Histogramm für ein Zielgebiet, beispielsweise ein Tumorgebiet, welches auch als Planning Target Volume (PTV) bezeichnet wird. Um eine homogene und hohe Bestrahlung des Zielgebiets zu erreichen, sollte der Graph 3 im Dosis-Volumen-Histogramm des Zielgebiets möglichst stufenförmig bei der gewünschten Zieldosis von 100% auf 0% abfallen. Bei einer idealen Stufe würde dies bedeuten, dass 100% des Zielgebiets die gewünschte Strahlendosis erhalten. In dem Dosis-Volumen-Histogramm der Fig. 1 weicht der Graph 3 geringfügig von einer idealen Stufenfunktion ab, d.h., ein Teil des Zielvolumens wird mit einer geringeren Strahlendosis als der gewünschten Strahlendosis von 1 Gy bestrahlt und ein anderer verhältnismäßig geringer Anteil des Zielvolumens wird mit einer höheren Strahlendosis belastet.

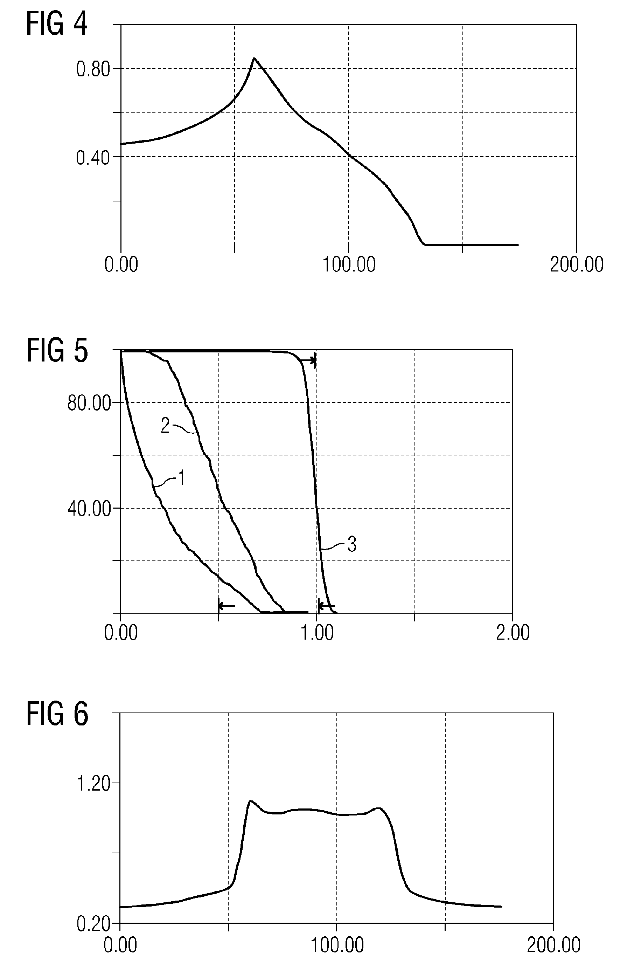

Fig. 1 zeigt das Dosis-Volumen-Histogramm, welches auf der Grundlage des IMPT-Verfahrens erstellt wurde. Der Benutzer hat als gewünschte Strahlendosis für das Zielgebiet 1Gy vorgegeben. In dem gezeigten Beispiel wird die Gesamtdosis für das Zielgebiet aus zwei Strahlen, die beispielsweise aus entgegengesetzten Richtungen auf das Zielgebiet einwirken, zusammengesetzt. Fig. 2 zeigt die Gesamtdosis über dem bestrahlten Gebiet, wobei auf der x-Achse eine Strecke in dem zu bestrahlenden Objekt oder Patienten aufgetragen ist und auf der y-Achse die dort erreichte Strahlendosis in Gray (Gy). In dem gewünschten Zielbereich (PTV) von beispielsweise 60mm-120mm wird eine verhältnismäßig homogene Gesamtdosis von gut 1Gy erreicht. Fig. 3 und 4 zeigen, wie sich die Gesamtdosis der Fig. 2 aus den beiden Strahlen zusammensetzt. Fig. 3 zeigt die Strahlendosis aufgrund eines ersten Strahls und Fig. 4 die Strahlendosis aufgrund eines zweiten Strahls. Hier ist eine starke Inhomogenität der einzelnen Strahlen in dem Zielgebiet (PTV) erkennbar. Der erste Strahl bewirkt eine sehr hohe Strahlendosis im Bereich zwischen 100 und 120 mm und der zweite Strahl bewirkt eine sehr hohe Strahlendosis im Bereich von 60 bis 80 mm. Derartig stark unterschiedliche Strahlendosen aufgrund einzelner Strahlen, d.h. eine hohe Strahleninhomogenität, ist jedoch unerwünscht, da die Gefahr besteht, dass insgesamt eine inhomogene Bestrahlung des Zielgebiets resultiert, wenn das Bestrahlungsobjekt zwischen der Bestrahlung mit dem ersten Strahl und der Bestrahlung mit dem zweiten Strahl umgelagert wird und dabei nicht ausreichend genau positioniert wird. Die Umpositionierung während der Behandlung ist nur eine mögliche Fehlerquelle. In der Teilchenbestrahlung kann auch eine globale Falschpositionierung eine Verschiebung der Dosisverteilungen gegeneinander bewirken. Eine andere Fehlerquelle ist, dass sich die Patientengeometrie zwischen Planung und Behandlung verändert (z.B. auf Grund einer Gewichtsabnahme). Darüber hinaus besteht die Gefahr, dass sensitives Gewebe, welches möglichst gering bestrahlt werden sollte, eine ungewünscht hohe Strahlungsdosis erhält, insbesondere nach einer nicht ausreichend genau positionierten Umlagerung des Bestrahlungsobjekts. Das herkömmliche IMPT-Verfahren erreicht somit zwar prinzipiell eine gute und homogene Bestrahlung des Zielbereichs, wie aus Fig. 1 und Fig. 2 ersichtlich ist, und schont dabei das sensitive gesunde Gewebe, ist jedoch sehr anfällig gegenüber Positionierungsfehlern und Patientenbewegungen während der Bestrahlung.

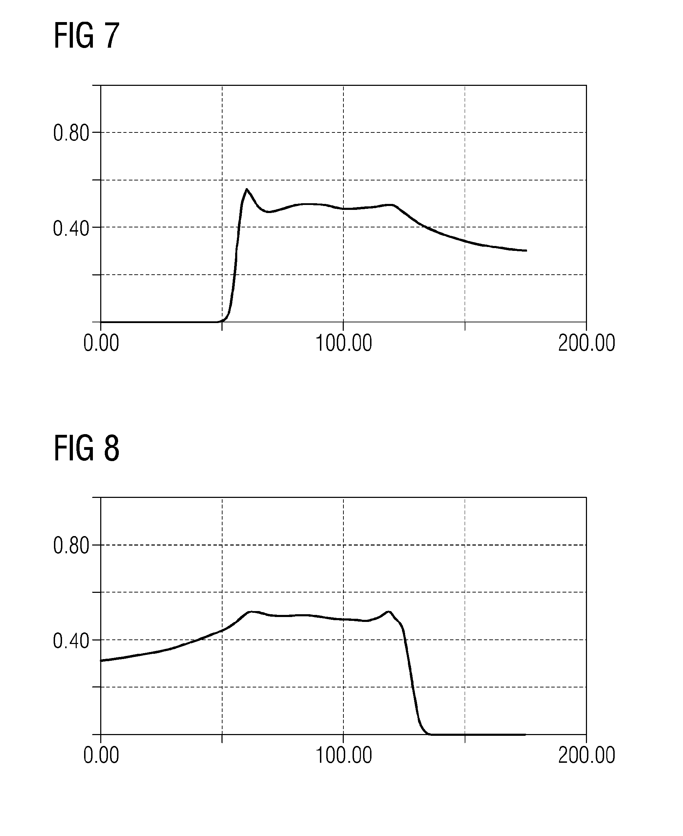

Fig. 5 zeigt ein Dosis-Volumen-Histogramm, welches mit Hilfe des SBO-Verfahrens erstellt wurde. Die Graphen 1 und 2 zeigen wiederum Bestrahlungsdosen für sensitives Gewebe im Strahlengang und Graph 3 die Strahlendosis für das Zielgebiet (PTV). Wie aus Fig. 5 insbesondere im Vergleich zu Fig. 1 ersichtlich ist, ist die gesamte Abdeckung des Zielgebiets mit einer gleichmäßigen Strahlendosis schlechter als bei dem IMPT-Verfahren der Fig. 1. Fig. 6 zeigt das gesamte Dosisprofil, welches durch zwei gegenüberliegende Strahlen mit den Strahlenprofilen der Fig. 7 und 8 erzeugt wird. Insbesondere der Vergleich der Fig. 7 und 8 mit den Fig. 3 und 4 zeigt, dass bei dem SBO-Verfahren eine höhere Einzelstrahlhomogenität erreicht werden kann, so dass das SBO-Verfahren robuster gegen Planungs- und Strahlzuführungsunsicherheiten oder Positionierungsungenauigkeiten bei einem Umlagern des Objekts zwischen einem Anwenden des ersten Strahls und des zweiten Strahls ist. Außerdem sind homogene Einzeldosen robuster gegenüber (initialen) Positionierungsfehlern.

In dem Stand der Technik sind daher Verfahren zur Optimierung von entweder dem IMPT-Verfahren oder dem SBO-Verfahren bekannt. Beispielsweise schlagen Martin Soukup et al. in "Study of Robustness of IMPT and IMRT for Prostate Cancer against Organ Movement" (Int J Radiat Oncol Biol Phys. 75(3):941-9 (2009)) ein Verfahren zur initialen Strahlengewichtseinstellung für ein IMPT-Verfahren vor, um bessere Startbedingungen für die Optimierung zu erhalten. Weiterhin schlagen F. Albertini et al. in "Degeneracy and Robustness of IMPT Plans in the Treatment of Skull-Base Chordomas" (Med. Phys. Volume 34, Issue 6, S. 2431-2431 (2007)) vor, das IMPT-Verfahren nur als Einstieg zu verwenden und somit nur einen Teil der gesamten Dosis über das IMPT-Verfahren zu liefern und den Großteil der Dosis über Strahlen, welche mit den SBO-Verfahren optimiert wurden, zu liefern.

Aufgabe der vorliegenden Erfindung ist es daher, verbesserte Verfahren zur Bestimmung eines Bestrahlungsplans für eine Bestrahlung eines Bestrahlungsobjekts mit mehreren Strahlen bereitzustellen.

Diese Aufgabe wird gemäß der vorliegenden Erfindung durch ein Verfahren zur Bestimmung eines Bestrahlungsplans für eine Bestrahlung eines Bestrahlungsobjekts nach Anspruch 1, eine Vorrichtung zur Bestimmung eines Bestrahlungsplans für eine Bestrahlung eines Bestrahlungsobjekts nach Anspruch 16, eine Partikelbestrahlungsanlage nach Anspruch 18, ein Computerprogrammprodukt nach Anspruch 19 und einen elektronisch lesbaren Datenträger nach Anspruch 20 gelöst. Die abhängigen Ansprüche definieren bevorzugte und vorteilhafte Ausführungsformen der Erfindung.

Gemäß der vorliegenden Erfindung wird ein Verfahren zur Bestimmung eines Bestrahlungsplans für eine Bestrahlung eines Bestrahlungsobjekts bereitgestellt. In dem Bestrahlungsobjekt sind mehrere Bestrahlungsbereiche festgelegt. Diese mehreren Bestrahlungsbereiche können beispielsweise Zielbereiche, auf welche eine besonders hohe Strahlungsdosis angewendet werden soll, und sensitive Bereiche, welche eine möglichst geringe Strahlendosis erhalten sollen, umfassen. Ein Bestrahlungsbereich kann auch ein einzelner Bildpunkt von beispielsweise einer Computertomographieaufnahme sein, welche als Grundlage einer Bestrahlungsplanung dient. Das Bestrahlungsobjekt wird mit mehreren Strahlen aus unterschiedlichen Richtungen bestrahlt. Die mehreren Strahlen können beispielsweise jeweils einzeln nacheinander auf das Bestrahlungsobjekt einwirken. Um die unterschiedlichen Strahlungsrichtungen zu erreichen, kann eine Richtung des Strahls geändert werden, das Bestrahlungsobjekt umgelagert werden oder sowohl die Strahlungsrichtung als auch die Lagerung des Bestrahlungsobjekts geändert werden. Bei dem Verfahren werden mehrere Gesamtdosisbedingungen bestimmt, wobei einem jeweiligen der mehreren Bestrahlungsbereiche jeweils mindestens eine der mehreren Gesamtdosisbedingungen zugeordnet wird. Die dem jeweiligen Bestrahlungsbereich zugeordnete Gesamtdosisbedingung legt eine Bedingung für die Gesamtstrahlendosis für den entsprechenden Bestrahlungsbereich fest. Somit wird jedem der mehreren Bereiche jeweils entweder eine Gesamtdosisbedingung, mehrere Gesamtdosisbedingungen oder keine Gesamtdosisbedingung zugeordnet. Weiterhin werden mehrere Einzelstrahldosisbedingungen bestimmt, wobei eine Einzelstrahldosisbedingung einem der mehreren Strahlen und der mehreren Bestrahlungsbereiche zugeordnet wird. Die dem jeweiligen Strahl zugeordnete Einzelstrahldosisbedingung legt eine Bedingung für die Strahlendosis aufgrund des Strahls für den jeweiligen Bestrahlungsbereich fest. In Abhängigkeit von den mehreren Gesamtdosisbedingungen und von den mehreren Einzelstrahldosisbedingungen werden Bestrahlungsparameter für die mehreren Strahlen bestimmt.

Indem zur Bestimmung der Bestrahlungsparameter für die mehreren Strahlen sowohl Gesamtdosisbedingungen als auch Einzelstrahldosisbedingungen berücksichtigt werden, können gewünschte Bestrahlungsdosen sowohl in dem Zielgebiet als auch in auszusparenden sensitiven oder gering zu bestrahlenden Gebieten genau eingestellt und erreicht werden, und gleichzeitig eine Homogenität der einzelnen Strahlen verbessert werden, wodurch der Bestrahlungsplan robuster gegenüber Planungs- und Strahlzuführungsunsicherheiten werden kann.

Gemäß einer Ausführungsform werden die Bestrahlungsparameter für die mehreren Strahlen mit Hilfe eines Optimierungsverfahrens bestimmt. Bei dem Optimierungsverfahren wird eine Zielfunktion in Abhängigkeit der Bestrahlungsparameter und der Bedingungen minimiert.

Gemäß einer weiteren Ausführungsform wird zum Bestimmen der mehreren Gesamtdosisbedingungen eine Benutzereingabe erfasst, welche den mehreren Strahlungsbereichen jeweils eine Gesamtdosisbedingung zuordnet.

Weiterhin können die Einzelstrahldosisbedingungen bestimmt werden, indem eine Benutzereingabe erfasst wird, welche den mehreren Strahlen für jeweils einen der Bestrahlungsbereiche jeweils eine Einzelstrahldosisbedingung zuordnet.

Indem die Gesamtdosisbedingungen und die Einzelstrahldosisbedingungen von einem Benutzer eingegeben werden können, ist eine sehr genaue und detaillierte Bestrahlungsplanung möglich.

Gemäß einer weiteren Ausführungsform werden die mehreren Einzelstrahldosisbedingungen automatisch in Abhängigkeit von einem vorgegebenen Strahlenhomogenitätsfaktor bestimmt. Der Strahlenhomogenitätsfaktor legt für einen der mehreren Bestrahlungsbereiche oder für alle Bestrahlungsbereiche eine maximale Abweichung zwischen den Strahlendosen, welche von den mehreren Strahlen in den Strahlungsbereich eingebracht werden, fest. In Abhängigkeit des Strahlenhomogenitätsfaktors können beispielsweise automatisch entsprechende Einzelstrahldosisbedingungen für die einzelnen Strahlen bestimmt werden. Der Strahlenhomogenitätsfaktor kann entweder fest vorgegeben sein oder über eine Benutzereingabe einstellbar sein.

Weiterhin kann über eine Benutzereingabe eine Strahlenhomogenitätsgewichtung erfasst werden, welche bei der Bestimmung der Bestrahlungsparameter verwendet wird. Die Strahlenhomogenitätsgewichtung gibt dabei ein Maß für die Berücksichtigung der Einzelstrahldosisbedingungen bei der Bestimmung der Bestrahlungsparameter vor. Die Bestrahlungsparameter für die mehreren Strahlen können wiederum durch Minimieren einer Zielfunktion optimiert werden. Die Zielfunktion ist dabei abhängig von dem Strahlenhomogenitätsfaktor bzw. den daraus abgeleiteten Einzelstrahldosisbedingungen, der Strahlenhomogenitätsgewichtung und den Gesamtdosisbedingungen.

Indem die Einzelstrahldosisbedingungen automatisch aus dem Strahlenhomogenitätsfaktor bestimmt werden, kann die Anzahl der von einem Benutzer einzugebenden Parameter bei der Bestimmung des Bestrahlungsplans erheblich verringert werden, so dass eine Bestimmung des Bestrahlungsplans schneller durchgeführt werden kann und eine weniger komplexe Aufgabe für den Benutzer darstellt. In Abhängigkeit der Strahlenhomogenitätsgewichtung kann der Benutzer auf einfache Art und Weise die Strahlenhomogenität je nach Anwendung erhöhen oder erniedrigen.

Gemäß einer Ausführungsform wird eine Dosisverteilung für einen Strahl auf der Grundlage eines vorgegebenen minimalen Gesamtdosispegels und eines vorgegebenen maximalen Gesamtdosispegels, des Homogenitätsfaktors und der Anzahl der Strahlen bestimmt. Dadurch können für jeden der Strahlen auf einfache Art und Weise entsprechende Einzelstrahldosisbedingungen bezüglich des Maximalwerts und des Minimalwerts für den Strahl aus dem Strahlenhomogenitätsfaktor bestimmt werden.

Bei einer weiteren Ausführungsform des Verfahrens wird ferner eine Benutzereingabe einer Einzelstrahlgewichtung erfasst. Die Bestrahlungsparameter werden für jeden der mehreren Strahlen in Abhängigkeit von den mehreren Gesamtdosisbedingungen, der mehreren Einzelstrahldosisbedingungen und der Einzelstrahlgewichtung bestimmt. Die Einzelstrahlgewichtung gibt ein Verhältnis zwischen einer Berücksichtigung der Einzelstrahldosisbedingungen und einer Berücksichtigung der Gesamtdosisbedingungen bei der Bestimmung der Bestrahlungsparameter vor. Dadurch wird einem Benutzer ermöglicht, je nach Anwendung die Einzelstrahldosisbedingungen oder die Gesamtdosisbedingungen stärker zu berücksichtigen, wodurch beispielsweise ein besseres Aussparen von nicht zu bestrahlendem gesunden Gewebe erreicht werden kann oder der Bestrahlungsplan insgesamt robuster gegen Planungs- und Zuführungsunsicherheiten gestaltet werden kann.

Gemäß einer weiteren Ausführungsform werden mehrere strahlspezifische Bestrahlungsbereiche in dem Bestrahlungsobjekt bestimmt und die Bestrahlungsparameter für die mehreren Strahlen in Abhängigkeit davon, ob sich die strahlspezifischen Bestrahlungsbereiche überlappen oder nicht, bestimmt. Für Bestrahlungsbereiche, in welchen sich die strahlspezifischen Bestrahlungsbereiche überlappen, werden die Bestrahlungsparameter in Abhängigkeit von den Gesamtdosisbedingungen für diese Bereiche bestimmt, wohingegen in Bestrahlungsbereichen, in welchen sich die strahlspezifischen Bestrahlungsbereiche nicht überlappen, die Bestrahlparameter in Abhängigkeit von den Einzelstrahldosisbedingungen bestimmt werden. Dadurch kann eine zuverlässige Aussparung von nicht zu bestrahlendem gesunden Gewebebereich sichergestellt werden, und gleichzeitig können Zielbereiche, welche nur von einem Strahl bestrahlt werden, zuverlässig bestrahlt werden.

Zusätzlich können beispielsweise benutzerspezifizierte Strahlgewichte den einzelnen Einzelstrahldosisbedingungen zugefügt werden, welche eine Berücksichtigung der entsprechenden Einzelstrahldosisbedingung bei der Optimierung des Bestrahlungsplans erhöhen oder herabsetzen.

Eine Gesamtstrahldosisbedingung kann beispielsweise in Form einer maximalen Dosis, einer minimalen Dosis, eines Dosis-Volumen-Histogramms, einer mittleren Dosis oder einer Strahlhomogenität angegeben werden. Eine Einzelstrahldosisbedingung kann beispielsweise in Form einer maximalen Dosis, einer minimalen Dosis oder einer mittleren Dosis vorgegeben werden.

Gemäß der vorliegenden Erfindung wird weiterhin eine Vorrichtung zur Bestimmung eines Bestrahlungsplans für eine Bestrahlung eines Bestrahlungsobjekts bereitgestellt. In dem Bestrahlungsobjekt sind mehrere Bestrahlungsbereiche festgelegt. Das Bestrahlungsobjekt wird mit mehreren Strahlen aus unterschiedlichen Richtungen bestrahlt. Die Vorrichtung umfasst eine erste Bestimmungsvorrichtung zum Bestimmen mehrerer Gesamtdosisbedingungen. Jeweils einem der mehreren Bestrahlungsbereiche ist jeweils mindestens eine der mehreren Gesamtdosisbedingungen zugeordnet. Die dem jeweiligen Bestrahlungsbereich zugeordnete Gesamtdosisbedingung legt eine Bedingung für die Gesamtstrahlendosis für den Bestrahlungsbereich fest. Weiterhin umfasst die Vorrichtung eine zweite Bestimmungsvorrichtung zum Bestimmen mehrere Einzelstrahldosisbedingungen. Einem jeweiligen der mehreren Strahlen und einem jeweiligen der mehreren Bestrahlungsbereiche ist jeweils eine der mehreren Einzelstrahldosisbedingungen zugeordnet. Die dem jeweiligen Strahl zugeordnete Einzelstrahldosisbedingung legt eine Bedingung für die Strahlendosis aufgrund des Strahls für den jeweiligen Bestrahlungsbereich fest. Schließlich umfasst die Vorrichtung eine Verarbeitungseinheit, welche in der Lage ist, Bestrahlungsparameter für die mehreren Strahlen in Abhängigkeit von den mehreren Gesamtdosisbedingungen und den mehreren Einzelstrahldosisbedingungen zu bestimmen.

Gemäß einer Ausführungsform ist die Vorrichtung derart ausgestaltet, dass sie zur Durchführung des zuvor beschriebenen Verfahrens und seiner Ausführungsformen geeignet ist und umfasst daher auch die zuvor beschriebenen Vorteile.

Die vorliegende Erfindung stellt weiterhin eine Partikelbestrahlungsanlage mit der zuvor beschriebenen Vorrichtung bereit, welche demzufolge ebenfalls die im Zusammenhang mit dem Verfahren beschriebenen Vorteile umfasst.

Des Weiteren stellt die vorliegende Erfindung ein Computerprogrammprodukt, insbesondere ein Computerprogramm oder eine Software, welche man in einen Speicher einer programmierbaren Verarbeitungseinheit einer Vorrichtung zur Bestimmung eines Bestrahlungsplans laden kann, bereit. Mit diesem Computerprogrammprodukt können alle oder verschiedene der zuvor beschriebenen Ausführungsformen des erfindungsgemäßen Verfahren ausgeführt werden, wenn das Computerprogrammprodukt in der Verarbeitungseinheit läuft. Dabei benötigt das Computerprogrammprodukt eventuell Programmmittel, zum Beispiel Bibliotheken und Hilfsfunktionen, um die entsprechenden Ausführungsformen des Verfahrens zu realisieren. Mit anderen Worten soll mit dem auf das Computerprogrammprodukt gerichteten Anspruch insbesondere ein Computerprogramm oder eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (zum Beispiel C++), welcher noch kompiliert (übersetzt) und gebunden oder welcher nur interpretiert werden muss, oder um einen ausführbaren Softwarecode handeln, welcher zur Ausführung nur noch in die entsprechende Verarbeitungseinheit zu laden ist.

Schließlich stellt die vorliegende Erfindung einen elektronisch lesbaren Datenträger, zum Beispiel eine DVD, ein Magnetband oder einen USB-Stick, bereit, auf welchem elektronisch lesbare Steuerinformationen, insbesondere Software, wie sie zuvor beschrieben wurde, gespeichert ist. Wenn diese Steuerinformationen bzw. Software von dem Datenträger gelesen und in der Verarbeitungseinheit gespeichert werden, können alle erfindungsgemäßen Ausführungsformen des beschriebenen Verfahrens durchgeführt werden.

Die Erfindung wird nachfolgend unter Bezugnahme auf die Zeichnungen anhand bevorzugter Ausführungsformen erläutert werden.

Fig. 1 zeigt ein Dosis-Volumen-Histogramm für einen Bestrahlungsplan, welcher mit Hilfe eines IMPT-Verfahrens erstellt wurde.

Fig. 2 zeigt das gesamte Dosisprofil für eine Bestrahlung, welche mit dem IMPT-Verfahren bestimmt wurde.

Fig. 3 zeigt ein Dosisprofil eines ersten Strahls einer Bestrahlung, welche mit dem IMPT-Verfahren bestimmt wurde.

Fig. 4 zeigt ein Dosisprofil eines zweiten Strahls einer Bestrahlung, welche mit dem IMPT-Verfahren bestimmt wurde.

Fig. 5-8 zeigen ein Dosis-Volumen-Histogramm, ein gesamtes Dosisprofil, ein Dosisprofil eines ersten Strahls und ein Dosisprofil eines zweiten Strahls für einen Bestrahlungsplan, welcher mit Hilfe eines SBO-Verfahrens erstellt wurde.

Fig. 9 zeigt eine Partikelbestrahlungsanlage gemäß einer Ausführungsform der vorliegenden Erfindung.

Fig. 10 zeigt ein Flussdiagramm mit den Schritten zur Bestimmung eines Bestrahlungsplans gemäß einer Ausführungsform der vorliegenden Erfindung.

Fig. 11 zeigt schematisch eine Durchführung eines Verfahrens zur Bestimmung eines Bestrahlungsplans gemäß einer Ausführungsform der vorliegenden Erfindung.

Fig. 12 zeigt schematisch eine Durchführung eines Verfahrens zur Bestimmung eines Bestrahlungsplans gemäß einer weiteren Ausführungsform der vorliegenden Erfindung.

Fig. 13 zeigt zwei Bedienelemente einer graphischen Benutzeroberfläche gemäß zweier Ausführungsformen der vorliegenden Erfindung.

Fig. 14 zeigt eine Schnittbildaufnahme eines Kopfes eines Patienten mit darin eingezeichneten Gebieten unterschiedlicher Strahlungsintensität.

Fig. 15-18 zeigen ein Dosis-Volumen-Histogramm, ein gesamtes Dosisprofil, ein Dosisprofil eines ersten Strahls und ein Dosisprofil eines zweiten Strahls, welche gemäß einer Ausführungsform der vorliegenden Erfindung bestimmt wurden.

Fig. 19-22 zeigen ein Dosis-Volumen-Histogramm, ein gesamtes Dosisprofil, eine Dosisprofil eines ersten Strahls und ein Dosisprofil eines zweiten Strahls gemäß einer weiteren Ausführungsform der vorliegenden Erfindung.

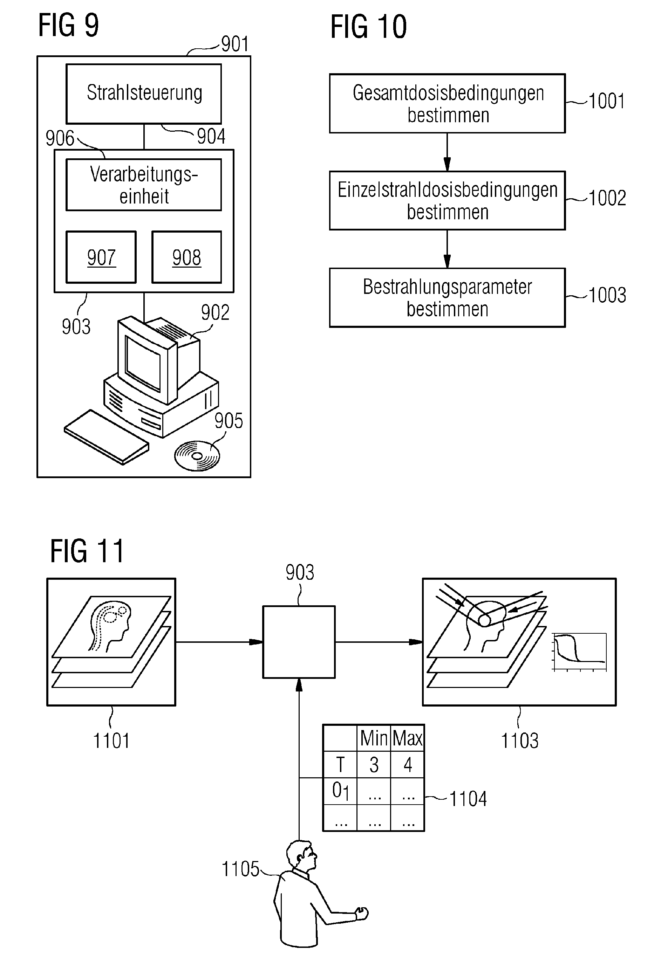

Fig. 9 zeigt eine Partikelbestrahlungsanlage 901, welche eine Benutzerschnittstelle 902, eine Vorrichtung zur Bestimmung eines Bestrahlungsplans 903 und eine Strahlsteuerung 904 umfasst. Die Benutzerschnittstelle 902 kann beispielsweise einen Bildschirm und eine Tastatur umfassen. Weiterhin kann die Benutzerschnittstelle 902 ein Lesegerät zum Lesen eines elektronisch lesbaren Datenträger 905 umfassen, auf welchem Programme für die Benutzerschnittstelle 902 und die Vorrichtung 903 gespeichert sind. Die Vorrichtung 903 umfasst eine Verarbeitungseinheit 906, eine erste Bestimmungseinheit 907 und eine zweite Bestimmungseinheit 908. Die Verarbeitungseinheit 906 und die Bestimmungseinheiten 907, 908 können auch als eine integrierte Einheit ausgebildet sein.

Unter Bezugnahme auf die Figuren 10 und 11 wird nachfolgend die Arbeitsweise der Partikelbestrahlungsanlage 901 näher beschrieben werden. Auf der Grundlage von Schichtbildern 1101, beispielsweise Magnetresonanzbildern oder Computertomographiebildern, werden Grundlagen eines Behandlungsplans bestimmt. Dabei werden die Schichtbilder 1101 beispielsweise in Bereiche segmentiert, welche Tumorbereiche und Organbereiche unterscheiden. Weiterhin wird die Anzahl der Strahlen und ihre jeweilige Richtung für die Bestrahlung des Tumorbereichs festgelegt. Weiterhin werden von einem Benutzer 1105 Bedingungen für den Bestrahlungsplan über die Benutzerschnittstelle 902 eingegeben. Diese Bedingungen können beispielsweise in Form einer Tabelle 1104 erfasst werden, worin für ausgewählte Bereiche Gesamtdosisbedingungen vorgegeben werden (Schritt 1001) und ausgewählten Strahlen für ausgewählte Bereiche Einzelstrahldosisbedingungen zugeordnet werden (Schritt 1002). In der in Fig. 11 gezeigten Tabelle 1104 werden beispielsweise für den Tumorbereich (T) jeweils ein Minimalwert (Min) und ein Maximalwert (Max) zugeordnet. Auch jedem weiteren Bereich, welcher bei der Segmentierung der Schichtbilder 1101 bestimmt wurde, werden entsprechende Bedingungen zugeordnet, beispielsweise einem Organbereich mit der Bezeichnung $O_1$. So können mehrere Tumorbereiche und mehrere Nicht-Tumorbereiche mit entsprechenden Gesamtdosisbedingungen und Einzelstrahldosisbedingungen versehen werden. Die Nicht-Tumorbereiche werden auch als sensitive Bereiche bezeichnet und sollten eine möglichst geringe Strahlendosis erhalten. Die Gesamtdosisbedingungen werden der Verarbeitungseinheit 906 über die erste Bestimmungseinheit 907 bereitgestellt und die Einzelstrahldosisbedingungen werden der Verarbeitungseinheit 906 über die zweite Bestimmungseinheit 908 bereitgestellt. Im Schritt 1003 werden von der Vorrichtung 903 mit Hilfe eines Optimierungsverfahrens auf der Grundlage der Gesamtdosisbedingungen, der Einzelstrahldosisbedingungen und der in den Schichtbildern 1101 definierten Bereiche Bestrahlungsparameter bestimmt. Auf der Grundlage der Bestrahlungsparameter kann ein Dosis-Volumen-Histogramm für die verschiedenen Bereiche simuliert werden und über die Benutzerschnittstelle 902 an einen Benutzer 1105 ausgegeben werden. Darüber hinaus kann ein Bestrahlungsplan 1103 auf der Grundlage der Bestrahlungsparameter erstellt werden und von einer Strahlsteuerung 904 bei einer Bestrahlung des Patienten verwendet werden.

Als Eingabe für das Optimierungsverfahren werden Dosisbedingungen verwendet, wobei jede Bedingung entweder auf die gesamte Dosis eines Bereichs oder auf eine der Einzelstrahldosen bezogen ist. Die gesamte Zielfunktion für die Optimierung der Bestrahlungsparameter p für die mehreren Strahlen lautet daher:

$$F(p) = F_{tot}(p) + \sum_i F_i(p)$$

Der erste Summand $F_{tot}(p)$ ist eine Bewertungsfunktion hinsichtlich einer Erfüllung der Gesamtdosisbedingungen bei den Bestrahlungsparametern p. Der zweite Summand ist eine Summe über alle Strahlen i und $F_i(p)$ ist eine Bewertungsfunktion hinsichtlich einer Erfüllung der Einzelstrahldosisbedingungen für den Strahl i bei den Bestrahlungsparametern p.

Die Funktion F(p) kann beispielsweise folgendermaßen über Abweichungen zu gewünschten Dosisbedingungen definiert werden:

$$F(p) = diff(D_{tot,desired}, D_{tot,act}(p)) + \sum_i (diff(D_{i,desired}, D_{i,act}(p)))$$

Für den ersten Summanden gibt der Benutzer Bedingungen bezüglich der Gesamtdosis vor. Beispielsweise stellt der Benutzer eine oder mehrere Bedingungen für bestimmte volumetrische Strukturen, zum Beispiel Organe oder den Zielbereich, ein. Die Bedingungen umfassen beispielsweise eine maximal erlaubte Dosis, eine minimal erlaubte Dosis, ein statistisch definiertes Dosis-Volumen-Histogramm oder eine gewünschte mittlere Dosis. Die gesamte Dosis kann daher auch eine Verteilungsfunktion der Dosis für die Struktur umfassen. In dem ersten Summanden bezeichnet p die Strahlparameter, Dtot,desired die gesamte gewünschte Dosis, $D_{tot,act(p)}$ die berechnete Dosis des aktuellen Optimierungsschritts und die Funktion diff() ein Maß einer Abweichung zwischen den beiden Dosen. $D_{tot,desired}$ ist implizit durch die eingestellten Dosenbedingungen definiert. Der zweite Summand ist eine Summe über alle Strahlen i. $D_{i,desired}$ wird implizit durch die eingestellten Einzelstrahldosisbedingungen definiert. $D_{i,act}$ ist die berechnete Dosis eines Strahls i für den aktuellen Optimierungsschritt. Die Funktion diff ( ) ist wiederum ein Maß für eine Abweichung zwischen den beiden Dosen. Die Zielfunktion F(p) kann beispielsweise iterativ minimiert werden. Darüber hinaus kann jede der zuvor genannten Bedingungen, also jede Gesamtdosenbedingung und jede Einzelstrahldosisbedingung, mit einem individuellen Gewicht versehen werden, welches es ermöglicht, eine relative Wichtigkeit der Einzelstrahldosisbedingung oder Gesamtdosisbedingung einzustellen. Der Benutzer kann insbesondere für jede Bedingung wählen, ob sie für die gesamte Dosis oder für eine der Einzelstrahldosen gilt.

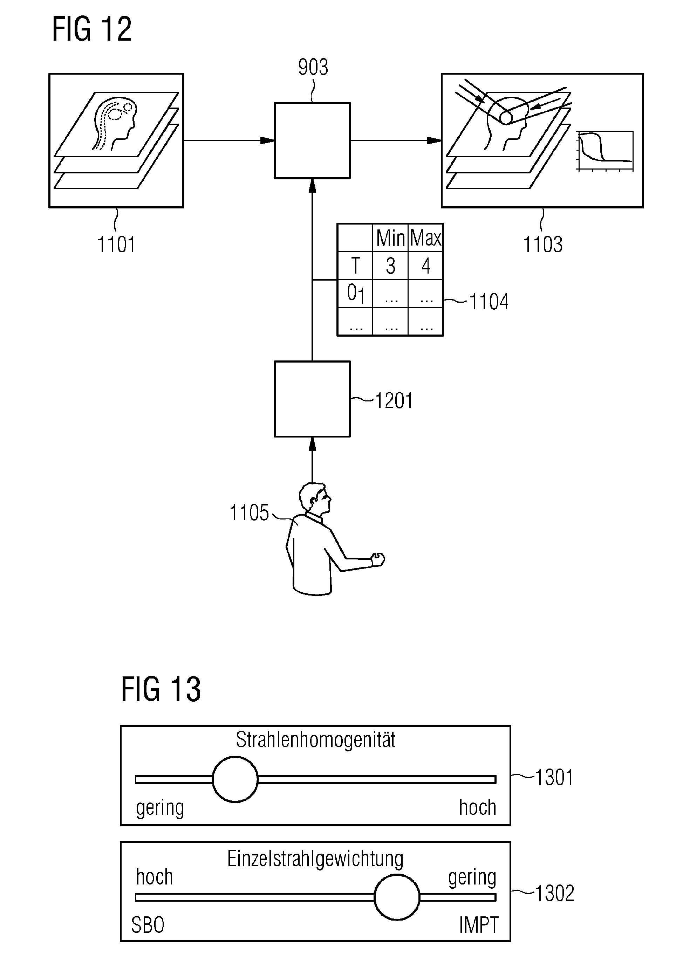

Diese manuelle Einstellen der Bedingungen ist eine verhältnismäßig komplexe Aufgabe für den Benutzer. Im Zusammenhang mit Fig. 12 wird daher eine Ausführungsform beschrieben, um diese Aufgabe zu vereinfachen. Das Erstellen der Gesamtdosisbedingungen und der Einzelstrahldosisbedingungen der Tabelle 1104 wird von einer weiteren Vorrichtung 1201, welche beispielsweise integriert mit der Verarbeitungseinheit 906 ausgebildet sein kann, durchgeführt. Dazu gibt der Benutzer 1105 in die Vorrichtung 1201 Bedingungen bezüglich der Gesamtdosis, beispielsweise eine Minimalbedingung und eine Maximalbedingung für das Zielvolumen, ein. Zusätzlich gibt der Benutzer zwei weitere Parameter ein. Der erste Parameter ist ein Homogenitätsfaktor HF, welcher beispielsweise im Bereich zwischen 0 und 1 gewählt wird. Der zweite Parameter ist eine Strahlenhomogenitätsgewichtung HW. Der Homogenitätsfaktor HF wird von der Vorrichtung 1201 in zusätzliche Bedingungen für die Einzelstrahldosen übersetzt. Die gesamte Zielfunktion F(p), die nunmehr zu minimieren ist, ergibt sich somit zu:

$$F(p) = F_{tot}(p) + HW \cdot \sum_i F_i(p, HF)$$

Dabei ist $F_{tot}(p)$ eine Bewertungsfunktion hinsichtlich einer Erfüllung der Gesamtdosisbedingungen bei den Bestrahlungsparametern p, und $F_i(p,HF)$ eine durch den Strahlenhomogenitätsfaktor HF bestimmte Bewertungsfunktion hinsichtlich einer Erfüllung der Einzelstrahldosisbedingungen für den Strahl i bei den Bestrahlungsparametern p.

Die Funktion F(p) kann beispielsweise wiederum folgendermaßen über Abweichungen zu gewünschten Dosisbedingungen definiert werden:

$$F(p) = diff(D_{tot,desired}, D_{tot,act}(p)) + HW \cdot \sum_i (diff(D_{i,desired}(HF), D_{i,act}(p)))$$

Als Einzelstrahldosisbedingungen $D_{i,desired}$ (HF) kann beispielsweise für jeden Strahl i eine Minimalbedingung $D_{min,i}$ und eine Maximalbedingung $D_{max,i}$ definiert und automatisch von der Vorrichtung 1201 bestimmt werden:

$$D_{max,i} = D_{max,tot} * (1 - HF/B)$$

$$D_{min,i} = D_{min,tot} * HF/B$$

wobei $D_{max,tot}$ und $D_{min,tot}$ die vom Benutzer vorgegebenen maximalen und minimalen Gesamtdosispegel für das Zielvolumen sind und $D_{max,i}$ und $D_{min,i}$ die abgeleiteten minimalen und maximalen Strahldosispegel für das Zielvolumen sind. B ist die Anzahl der an dem Zielvolumen beteiligten Strahlen. Für Bereiche außerhalb des Zielbereichs, also die sensitiven Bereiche, gelten nur die Bedingungen der Gesamtdosis. Als eine Erweiterung dieser Ausführungsform können benutzereinstellbare Strahlgewichte in den obigen Gleichungen verwendet werden. Bei einer Gesamtzahl von zwei Strahlen (B=2) und $D_{max,tot}=D_{min,tot}$ und HF=1 wird für jeden Strahl gefordert, 50% der gewünschten Zieldosis zu liefern. Bei ansonsten gleicher Einstellung und HF=0,5 wird für jeden Strahl gefordert, nicht mehr als 75% und nicht weniger als 25% der gewünschten Zieldosis zu liefern. Wenn HF gleich Null gesetzt wird, ergeben sich keine zusätzlichen Bedingungen für die Einzelstrahldosen.

Die Strahlenhomogenitätsgewichtung HW ermöglicht es, die Wirkung der Einzelstrahldosisbedingungen zu gewichten. Mit HW=0 ergibt sich keine Wirkung der Bedingungen auf die Einzelstrahldosen.

Im Vergleich zu dem in Fig. 11 beschriebenen Verfahren kann der Benutzer bei dem in Fig. 12 beschriebenen Verfahren keine unterschiedlichen Anforderungen für jeden Strahl einstellen und somit keine feingranularen Bedingungen wählen. Dafür wird die Konfiguration insgesamt einfacher und die Strahlenhomogenitätsgewichtung HW ermöglicht es dem Benutzer, eine globale Steuerung der Beeinflussung der Einzelstrahldosisbedingungen durchzuführen.

Alternativ kann der Homogenitätsfaktor HF auch vom System fest vorgegeben sein.

Gemäß einer weiteren Ausführungsform kann der Benutzer, wie zuvor beschrieben wurde, die Gesamtdosisbedingungen und die Einzelstrahldosisbedingungen einstellen und eine relative Einzelstrahlengewichtung W einstellen. W kann beispielsweise im Bereich von 0 bis 1 einstellbar sein. Die Zielfunktion F(p), anhand derer durch Minimieren die Strahlungsparameter p für die mehreren Strahlen bestimmt werden, lautet in diesem Fall:

$$F(p) = (1-W) \cdot F_{tot}(p) + W \cdot \sum_i F_i(p)$$

Dabei ist $F_{tot}(p)$ eine Bewertungsfunktion hinsichtlich einer Erfüllung der Gesamtdosisbedingungen bei den Bestrahlungsparametern p, und $F_i(p)$ eine Bewertungsfunktion hinsichtlich einer Erfüllung der Einzelstrahldosisbedingungen für den Strahl i bei den Bestrahlungsparametern p.

Die Funktion F(p) kann beispielsweise wiederum folgendermaßen über Abweichungen zu gewünschten Dosisbedingungen definiert werden:

$$F(p) = (1-W) \cdot diff(D_{tot,desired}, D_{tot,act}(p)) + W \cdot \sum_i (diff(D_{i,desired}, D_{i,act}(p)))$$

Somit können über die Einzelstrahlgewichtung W entweder die Einzelstrahldosisbedingungen (zweiter Summand) oder die Gesamtdosisbedingungen (erster Summand) stärker berücksichtigt werden. Der erste Summand entspricht dabei einer Optimierungsstrategie wie bei einem IMPT-Verfahren und der zweite Summand entspricht einer Optimierungsstrategie wie bei einem SBO-Verfahren.

Fig. 13 zeigt graphische Bedienelemente der Benutzerschnittstelle 902 zur Einstellung des Strahlenhomogenitätsfaktors HF mit Hilfe eines Schiebers 1301 und zur Einstellung der Einzelstrahlgewichtung W mit Hilfe eines Schiebers 1302.

Bei einer weiteren Ausführungsform kann der Benutzer für jeden Strahl ein eigenes Zielgebiet definieren. Diese Zielgebiete sind weitestgehend überlappend aber unterscheiden sich dennoch geringfügig. Der Benutzer kann, wie zuvor beschrieben wurde, Gesamtdosisbedingungen für das Zielgebiet und normale Organe oder sensitive Bereiche definieren. Auf der Grundlage dieser Information werden von der Vorrichtung 903 lokale Dosisbedingungen folgendermaßen erzeugt. In Bereichen, in denen sich die Zielgebiete überschneiden, werden die Gesamtdosisbedingungen angewendet, und in Bereichen, welche nur für einen Strahl i relevant sind, werden Einzelstrahlbedingungen für den Strahl i angewendet, mit $D_i=D_{target}/B$. Optional kann der Benutzer die Einzelstrahlbedingungen gewichten. In Bereichen mit normalen Organen oder sensitiven Bereichen werden die Gesamtdosisbedingungen angewendet. Dadurch werden die Vorteile eines IMPT-Verfahrens mit den Vorteilen von strahlabhängigen Zielbereichen kombiniert.

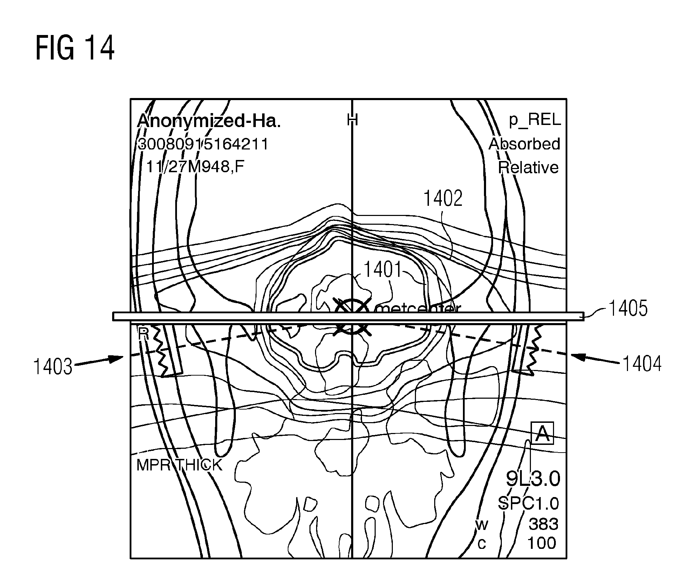

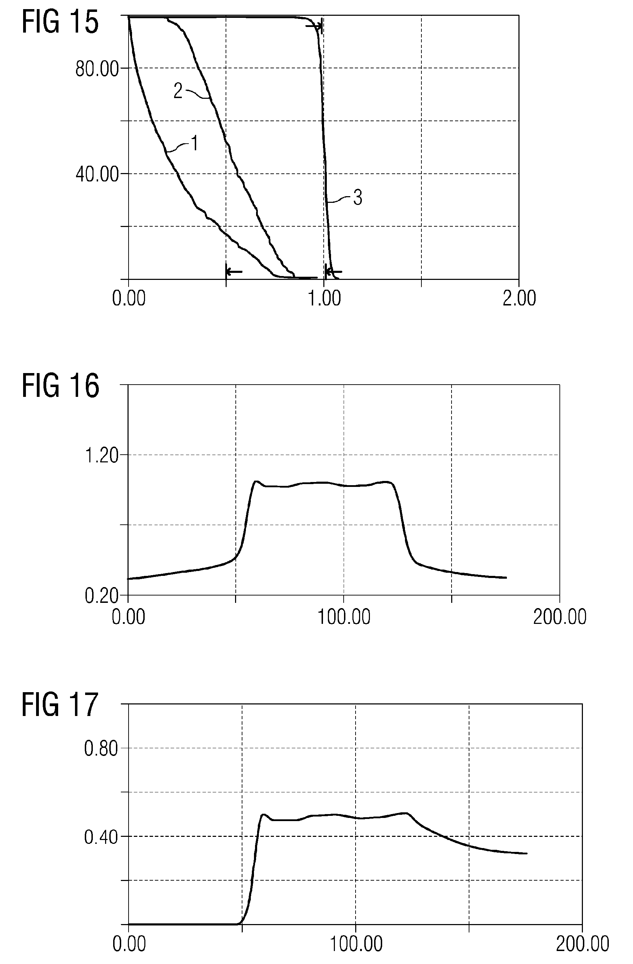

Fig. 14 zeigt eine Schichtbildaufnahme eines Kopfes. In der Mitte der Aufnahme befindet sich das Zielgebiet, beispielsweise ein Tumor. Die eingezeichneten Linienzüge begrenzen Gebiete gleicher Strahlungsintensität. Beispielhaft sind die Linienzüge 1401 und 1402 mit Bezugszeichen gekennzeichnet. Der Kopf wird von zwei Partikelstrahlen in Richtung der Pfeile 1403 und 1404 bestrahlt. Weiterhin ist eine Schnittlinie 1405 eingezeichnet, welche unter Bezugnahme auf die Fig. 15-22 verwendet wird.

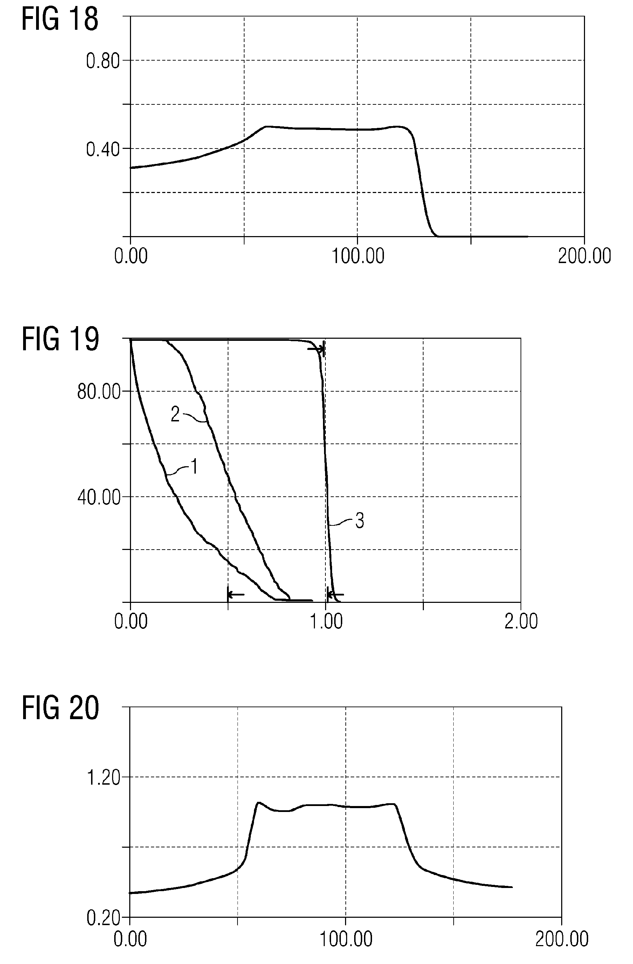

Fig. 15 zeigt ein Dosis-Volumen-Histogramm für einen Bestrahlungsplan, welcher gemäß der im Zusammenhang mit Fig. 12 beschriebenen Ausführungsform bestimmt wurde. Der Homogenitätsfaktor wurde zu HF=1 und die Strahlenhomogenitätsgewichtung zu HW=1 gesetzt. Deutlich zu erkennen ist in Fig. 15 die steile Stufe des Graphen 3 für das Zielgebiet bzw. den Tumor. Die sensitiven Organe werden, wie mit den Graphen 1 und 2 gezeigt wird, erheblich geringer belastet. Der Homogenitätsfaktor HF=1 strebt eine gleichmäßige Intensitätsverteilung bei den einzelnen Strahlen an. Fig. 16 zeigt das gesamte Dosisprofil und Fig. 17 und 18 die Dosisprofile der Strahlen 1404 und 1403. Deutlich zu erkennen ist in den Fig. 17 und 18 die homogene Dosisverteilung zwischen den Strahlen 1404 und 1403. Jeder der beiden Strahlen trägt, wie mit HF=1 gefordert, näherungsweise 50% der gesamten Strahlendosis bei.

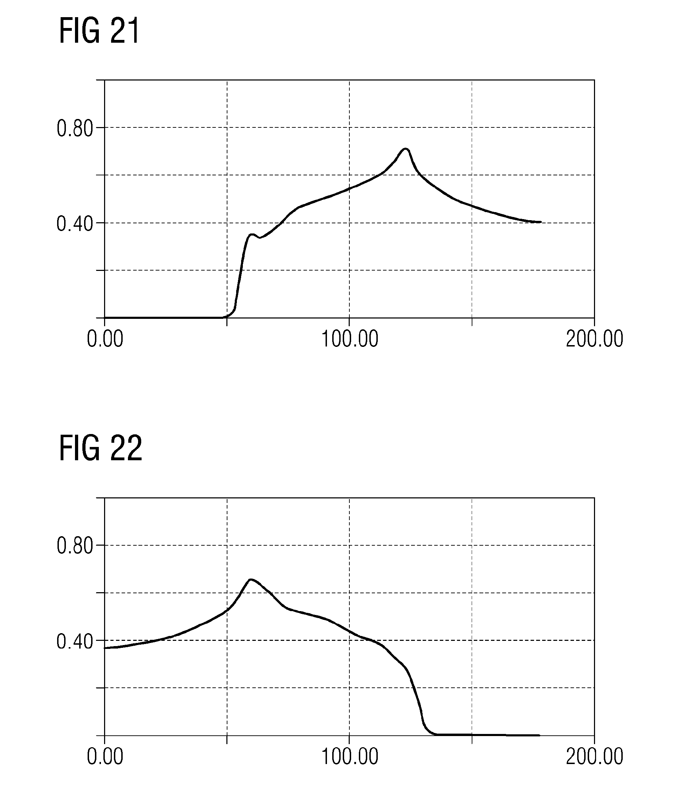

Fig. 19 zeigt ein Dosis-Volumen-Histogramm für einen Homogenitätsfaktor von HF=0,6 und eine Strahlenhomogenitätsgewichtung von HW=1. Im Vergleich zu dem Dosis-Volumen-Histogramm der Fig. 15 werden in Fig. 19 die sensitiven Bereiche weniger stark belastet, wie aus der Lage der Grahpen 1 und 2 ersichtlich ist. Das Gesamtdosisprofil, welches in Fig. 20 gezeigt ist, ist vergleichbar zu dem in Fig. 16. Das Dosisprofil des Strahls 1404 ist in Fig. 21 gezeigt und das Dosisprofil des Strahls 1403 ist in Fig. 22 gezeigt. Fig. 21 und 22 zeigen im Vergleich zu den Fig. 17 und 18 eine deutlich erhöhte Strahlinhomogenität auf. Allerdings überschreiten die Einzeldosen nicht einen Korridor von 30%-70%. Dies wird durch den Homogenitätsfaktor HF=0,6 sichergestellt.

Der Benutzer kann somit die Einzelstrahldosisverteilungen steuern und gleichzeitig die Vorteile eines IMPT-Verfahrens nutzen. Darüber hinaus kann der Benutzer strahlabhängige Zielbereiche definieren. Wie aus den Fig. 15-22 ersichtlich ist, kann eine sehr gute Zielabdeckung erreicht werden und gleichzeitig eine gewünschte Homogenität der Einzelstrahldosen erreicht werden.

Obwohl die vorliegende Erfindung in der vorhergehenden Beschreibung im Wesentlichen im Zusammenhang mit einer Bestrahlungsplanung einer Partikeltherapie beschrieben wurde, sind die Verfahren der vorliegenden Erfindung jedoch auch gleichermaßen für eine Bestimmung eines Bestrahlungsplan für eine allgemeine Strahlentherapie mit beispielsweise Gammastrahlung, Photonenstrahlung oder Röntgenstrahlung geeignet.

Bezugszeichenliste

| 1 | Graph |
| 2 | Graph |
| 3 | Graph |
| 901 | Partikelbestrahlungsanlage |
| 902 | Benutzerschnittstelle |
| 903 | Vorrichtung |
| 904 | Strahlsteuerung |
| 905 | Datenträger |
| 906 | Verarbeitungseinheit |
| 907 | Bestimmungseinheit |
| 908 | Bestimmungseinheit |
| 1001 | Schritt |
| 1002 | Schritt |
| 1003 | Schritt |
| 1101 | Schichtbildern |
| 1103 | Bestrahlungsplan |
| 1104 | Tabelle |
| 1105 | Benutzer |
| 1201 | Vorrichtung |
| 1301 | Schieber |
| 1302 | Schieber |
| 1401 | Linienzug |
| 1402 | Linienzug |
| 1403 | Pfeil |
| 1404 | Pfeil |
| 1405 | Schnittlinie |

Patentansprüche

1. Verfahren zur Bestimmung eines Bestrahlungsplans für eine Bestrahlung eines Bestrahlungsobjekts, wobei in dem Bestrahlungsobjekt mehrere Bestrahlungsbereiche festgelegt sind, wobei das Bestrahlungsobjekt mit mehreren Strahlen (1403, 1404) aus unterschiedlichen Richtungen bestrahlt wird, umfassend:

- Bestimmen mehrerer Gesamtdosisbedingungen, wobei einem jeweiligen der mehreren Bestrahlungsbereiche jeweils mindestens eine der mehreren Gesamtdosisbedingungen zugeordnet ist, wobei die dem jeweiligen Bestrahlungsbereich zugeordnete Gesamtdosisbedingung eine Bedingung für die Gesamtstrahlendosis für den Bestrahlungsbereich festlegt,

- Bestimmen mehrerer Einzelstrahldosisbedingungen, wobei einem jeweiligen der mehreren Strahlen (1403, 1404) und einem jeweiligen der mehreren Bestrahlungsbereiche jeweils mindestens eine der mehreren Einzelstrahldosisbedingungen zugeordnet ist, wobei die dem jeweiligen Strahl (1403, 1404) zugeordnete Einzelstrahldosisbedingung eine Bedingung für die Strahlendosis aufgrund des Strahls (1403, 1404) für den jeweiligen Bestrahlungsbereich festlegt, und

- Bestimmen von Bestrahlungsparametern für die mehreren Strahlen (1403, 1404) in Abhängigkeit von den mehreren Gesamtdosisbedingungen und von den mehreren Einzelstrahldosisbedingungen,

**dadurch gekennzeichnet, dass**

das Bestimmen der mehreren Einzelstrahldosisbedingungen ein automatisches Bestimmen mehrerer Einzelstrahldosisbedingungen in Abhängigkeit von einem vorgegebenen Strahlenhomogenitätsfaktor HF umfasst, wobei der Strahlenhomogenitätsfaktor HF für einen der mehreren Bestrahlungsbereiche eine maximale Abweichung zwischen von den mehreren Strahlen (1403, 1404) in den Bestrahlungsbereich eingebrachten Strahlendosen festlegt.

2. Verfahren nach Anspruch 1, wobei das Bestimmen der Bestrahlungsparameter p für die mehreren Strahlen (1403, 1404) ein Minimieren einer Zielfunktion F(p) mit

$$F(p) = F_{tot}(p) + \sum_i F_i(p)$$

umfasst, wobei

$F_{tot}(p)$ eine Bewertungsfunktion hinsichtlich einer Erfüllung der Gesamtdosisbedingungen bei den Bestrahlungsparametern p umfasst, und

$F_i(p)$ eine Bewertungsfunktion hinsichtlich einer Erfüllung der Einzelstrahldosisbedingungen für den Strahl i bei den Bestrahlungsparametern p umfasst.

3. Verfahren nach Anspruch 1 oder 2, wobei das Bestimmen der mehreren Gesamtdosisbedingungen ein Erfassen einer Benutzereingabe (1104) einer jeweiligen Gesamtdosisbedingung für jeweils einen der mehreren Bestrahlungsbereiche umfasst.

4. Verfahren nach einem der vorhergehenden Ansprüche, wobei das Bestimmen mehrerer Einzelstrahldosisbedingungen ein Erfassen einer Benutzereingabe (1104) einer jeweiligen Einzelstrahldosisbedingung für jeweils einen der mehreren Strahlen (1403, 1404) für jeweils einen der mehreren Bestrahlungsbereiche umfasst.

5. Verfahren nach einem der vorhergehenden Ansprüche, wobei das Verfahren ferner ein Erfassen einer Benutzereingabe (1301) umfasst, mit welcher ein Benutzer den Strahlenhomogenitätsfaktor HF vorgibt.

6. Verfahren nach einem der vorhergehenden Ansprüche, wobei der Strahlenhomogenitätsfaktor HF einen fest vorgegeben Wert umfasst.

7. Verfahren nach einem der vorhergehenden Ansprüche, wobei das Verfahren ferner ein Erfassen einer Benutzereingabe einer Strahlenhomogenitätsgewichtung HW umfasst,
wobei das Bestimmen der Bestrahlungsparameter ein Bestimmen von Bestrahlungsparametern für jeden der mehreren Strahlen (1403, 1404) in Abhängigkeit von den mehreren Gesamtdosisbedingungen, den mehreren Einzelstrahldosisbedingungen und der Strahlenhomogenitätsgewichtung HW umfasst,
wobei die Strahlenhomogenitätsgewichtung HW ein Maß für die Berücksichtigung der Einzelstrahldosisbedingungen bei der Bestimmung der Bestrahlungsparameter festlegt.

8. Verfahren nach Anspruch 7, wobei das Bestimmen der Bestrahlungsparameter p für die mehreren Strahlen ein Minimieren einer Zielfunktion F(p) mit

$$F(p) = F_{tot}(p) + HW \cdot \sum_i F_i(p, HF)$$

umfasst, wobei

$F_{tot}(p)$ eine Bewertungsfunktion hinsichtlich einer Erfüllung der Gesamtdosisbedingungen bei den Bestrahlungsparametern p umfasst, und

$F_i(p,HF)$ eine durch den Strahlenhomogenitätsfaktor HF bestimmte Bewertungsfunktion hinsichtlich einer Erfüllung der Einzelstrahldosisbedingungen für den Strahl i bei den Bestrahlungsparametern p umfasst.

9. Verfahren nach Anspruch 8, wobei die durch den Strahlenhomogenitätsfaktor HF bestimmte Bewertungsfunktion $F_i(p,HF)$ für den Strahl i auf der Grundlage eines vorgegebenen minimalen Gesamtdosispegels $D_{min,tot}$ und eines vorgegebenen maximalen Gesamtdosispegels $D_{max,tot}$ bestimmt wird, wobei die Dosisbewertungsfunktion für den Strahl i über einen maximalen Dosispegel $D_{max,i}$ für den Strahl i und einen minimalen Dosispegel $D_{min,i}$ für den Strahl i gemäß der Gleichungen:

$$D_{max,i} = D_{max,tot} * (1 - HF / B)$$

und

$$D_{min,i} = D_{min,tot} * HF / B$$

bestimmt wird, wobei B die Anzahl der Strahlen (1403, 1404) ist.

10. Verfahren nach einem der vorhergehenden Ansprüche, wobei das Verfahren ferner ein Erfassen einer Benutzereingabe (1302) einer Einzelstrahlengewichtung W umfasst,

wobei das Bestimmen der Bestrahlungsparameter ein Bestimmen von Bestrahlungsparametern für jeden der mehreren Strahlen (1403, 1404) in Abhängigkeit von den mehreren Gesamtdosisbedingungen, den mehreren Einzelstrahldosisbedingungen und der Einzelstrahlengewichtung umfasst,

wobei die Einzelstrahlengewichtung W ein Verhältnis zwischen einer Berücksichtigung der Einzelstrahldosisbedingungen und einer Berücksichtigung der Gesamtdosisbedingungen bei der Bestimmung der Bestrahlungsparameter festlegt.

11. Verfahren nach Anspruch 10, wobei das Bestimmen der Bestrahlungsparameter p für die mehreren Strahlen ein Minimieren einer Zielfunktion F(p) mit

$$F(p) = (1 - W) \cdot F_{tot}(p) + W \cdot \sum_i F_i(p)$$

umfasst, wobei

$F_{tot}(p)$ eine Bewertungsfunktion hinsichtlich einer Erfüllung der Gesamtdosisbedingungen bei den Bestrahlungsparametern p umfasst, und

$F_i(p)$ eine Bewertungsfunktion hinsichtlich einer Erfüllung der Einzelstrahldosisbedingungen für den Strahl i bei den Bestrahlungsparametern p umfasst.

12. Verfahren nach einem der vorhergehenden Ansprüche, ferner umfassend ein Bestimmen mehrerer strahlspezifischer Bestrahlungsbereiche in dem Bestrahlungsobjekt,

wobei die Bestrahlungsparameter für die mehreren Strahlen (1403, 1404) für Bestrahlungsbereiche, in welchen sich die strahlspezifischen Bestrahlungsbereiche überlappen, in Abhängigkeit von den Gesamtdosisbedingungen bestimmt werden und für Bestrahlungsbereiche, in welchen sich die strahlspezifischen Bestrahlungsbereiche nicht überlappen, in Abhängigkeit von den Einzelstrahldosisbedingungen bestimmt werden.

13. Verfahren nach einem der vorhergehenden Ansprüche, wobei die Gesamtdosisbedingung eine maximale Dosis, eine minimale Dosis, ein Dosis-Volumen-Histogramm, eine mittlere Dosis, eine Äquivalent-Uniforme Dosis (EUD), eine biologische Zielgröße oder eine Dosishomogenität umfasst.

**14.** Verfahren nach einem der vorhergehenden Ansprüche, wobei die Einzelstrahldosisbedingung eine maximale Dosis, eine minimale Dosis oder eine mittlere Dosis oder eine Dosishomogenität umfasst.

**15.** Vorrichtung zur Bestimmung eines Bestrahlungsplans für eine Bestrahlung eines Bestrahlungsobjekts, wobei in dem Bestrahlungsobjekt mehrere Bestrahlungsbereiche festgelegt sind, wobei das Bestrahlungsobjekt mit mehreren Strahlen (1403, 1404) aus unterschiedlichen Richtungen bestrahlt wird, umfassend:

- eine erste Bestimmungseinheit (907) zum Bestimmen mehrerer Gesamtdosisbedingungen, wobei einem jeweiligen der mehreren Bestrahlungsbereiche jeweils mindestens eine der mehreren Gesamtdosisbedingungen zugeordnet ist, wobei die dem jeweiligen Bestrahlungsbereich zugeordnete Gesamtdosisbedingung eine Bedingung für die Gesamtstrahlendosis für den Bestrahlungsbereich festlegt,
- eine zweite Bestimmungseinheit (908) zum Bestimmen mehrerer Einzelstrahldosisbedingungen, wobei einem jeweiligen der mehreren Strahlen (1403, 1404) und einem jeweiligen der mehreren Bestrahlungsbereiche jeweils mindestens eine der mehreren Einzelstrahldosisbedingungen zugeordnet ist, wobei die dem jeweiligen Strahl (1403, 1404) zugeordnete Einzelstrahldosisbedingung eine Bedingung für die Strahlendosis aufgrund des Strahls (1403, 1404) für den jeweiligen Bestrahlungsbereich festlegt, und
- eine Verarbeitungseinheit (906), welche ausgestaltet ist, Bestrahlungsparameter für die mehreren Strahlen (1403, 1404) in Abhängigkeit von den mehreren Gesamtdosisbedingungen und den mehreren Einzelstrahldosisbedingungen zu bestimmen, **dadurch gekennzeichnet, dass** die zweite Bestimmungseinheit (908) derart eingerichtet ist, dass
das Bestimmen der mehrerer Einzelstrahldosisbedingungen ein automatisches Bestimmen mehrerer Einzelstrahldosisbedingungen in Abhängigkeit von einem vorgegebenen Strahlenhomogenitätsfaktor HF umfasst, wobei der Strahlenhomogenitätsfaktor HF für einen der mehreren Bestrahlungsbereiche eine maximale Abweichung zwischen von den mehreren Strahlen (1403, 1404) in den Bestrahlungsbereich eingebrachten Strahlendosen festlegt.

**16.** Vorrichtung nach Anspruch 15, **dadurch gekennzeichnet, dass** die Vorrichtung (903) zur Durchführung des Verfahrens nach einem der Ansprüche 2-14 ausgestaltet ist.

**17.** Partikelbestrahlungsanlage mit einer Vorrichtung (903) nach Anspruch 15 oder 16.

**18.** Computerprogrammprodukt, welches ein Programm umfasst und direkt in einen Speicher einer programmierbaren Verarbeitungseinheit (906) einer Vorrichtung (903) zur Bestimmung eines Bestrahlungsplans ladbar ist, mit Programm-Mitteln, um alle Schritte des Verfahrens nach einem der Ansprüche 1-14 auszuführen, wenn das Programm in der Verarbeitungseinheit (906) ausgeführt wird.

**19.** Elektronisch lesbarer Datenträger mit darauf gespeicherten elektronisch lesbaren Steuerinformationen, welche derart ausgestaltet sind, dass sie bei Verwendung des Datenträgers (905) in einer Verarbeitungseinheit (906) einer Vorrichtung (903) zur Bestimmung eines Bestrahlungsplans das Verfahren nach einem der Ansprüche 1-14 durchführen.

**Claims**

**1.** Method for determining a radiotherapy treatment plan for an irradiation of an object to be irradiated, wherein a number of irradiation areas are determined in the object to be irradiated, whereby the object to be irradiated is irradiated with a number of beams (1403, 1404) from different directions, comprising

- Determining a number of total dose conditions, wherein one of the number of irradiation areas in each case is assigned at least one of the number of total dose conditions in each case, wherein the total dose condition assigned to the respective irradiation area defines a condition for the total radiation dose for the irradiation area,
- Determining a number of single beam dose conditions, wherein one of the number of beams (1403, 1404) in each case and one of the number of irradiation areas in each case is assigned one of the number of single beam dose conditions in each case, wherein the single beam dose condition assigned to the respective beam (1403, 1404) defines a condition for the beam dose as a result of the beam (1403, 1404) for the respective irradiation area, and
- Determining irradiation parameters for the number of beams (1403, 1404) as a function of the number of total dose conditions and of the number of single beam dose conditions, **characterised in that**

the determination of the number of single beam dose conditions includes an automatic determination of a number of single beam dose conditions as a function of a predetermined beam homogeneity factor HF, wherein the beam homogeneity factor HF defines for one of the number of irradiation areas a maximum difference between radiation doses introduced by the number of beams (1403, 1404) into the irradiation area.

**2.** Method according to claim 1, wherein the determination of the irradiation parameters p for the number of beams (1403, 1404) also includes a minimisation of a target function F(p),

$$F(p) = F_{tot}(p) + \sum_i F_i(p)$$

wherein
$F_{tot}(p)$ includes an evaluation function in respect of a fulfillment of the total dose conditions with the irradiation parameters p, and
$F_i(p)$ includes an evaluation function in respect of a fulfillment of the single beam dose conditions for the beam i with the irradiation parameters p.

**3.** Method according to claim 1 or 2, with the determination of the number of total dose conditions comprising capturing of a user input (1104) of a respective total dose condition for one of the number of irradiation areas in each case.

**4.** Method according to one of the preceding claims, wherein the determination of a number of single beam dose conditions includes capturing of a user input (1104) of a respective single beam dose condition for one of the number of beams (1403, 1404) in each case for one of the number of the irradiation areas in each case.

**5.** Method according to one of the preceding claims, wherein the method further includes capturing of a user input (1301) with which a user specifies the beam homogeneity factor HF.

**6.** Method according to one of the preceding claims, with the beam homogeneity factor HF comprising a fixed specified value.

**7.** Method according to one of the preceding claims, with the method further including the capturing of a user input of a beam homogeneity weighting HW,
wherein the determination of the irradiation parameters comprises determining irradiation parameters for each of the number of beams (1403, 1404) as a function of the number of total dose conditions, the number of single beam dose conditions and the beam homogeneity weighting HW,
wherein the beam homogeneity weighting HW defines a measure for taking account of the single dose conditions in the determination of the irradiation parameters.

**8.** Method according to claim 7, wherein the determination of the irradiation parameters p for the number of beams comprises a minimisation of a target function F(p) with

$$F(p) = F_{tot}(p) + HW \cdot \sum_i F_i(p, HF)$$

wherein
$F_{tot}(p)$ includes an evaluation function in respect of fulfillment of the total dose conditions for the irradiation parameters p, and
$F_i(p, HF)$ includes an evaluation function determined by the beam homogeneity factor HF in respect of a fulfillment of the single beam dose conditions for the beam i for the irradiation parameters p.

**9.** Method according to claim 8, wherein the evaluation function $F_i(p, HF)$ determined by the beam homogeneity factor HF for the beam i is determined on the basis of a predetermined minimum total dose level $D_{min,tot}$ and a predetermined maximum total dose level $D_{max,tot}$, wherein the dose evaluation function for the beam i is determined via a maximum dose level $D_{max,i}$ for the beam i and a minimum dose level $D_{min,i}$ for the beam i in accordance with the equations:

$$D_{\max,i} = D_{\max,tot} * (1 - HF / B)$$

and

$$D_{\min,i} = D_{\min,tot} * HF / B$$

, wherein B is the number of beams (1403, 1404).

10. Method according to one of the previous claims, wherein the method further comprises detection of a user input (1302) of a single beam weighting W,
wherein the determination of the irradiation parameters comprises a determination of irradiation parameters for each of the number of beams (1403, 1404) as a function of the number of total dose conditions, the number of single beam dose conditions and the single beam weighting,
wherein the single beam weighting W defines a relationship between taking account of the single beam dose conditions and taking account of the total dose conditions in the determination of the irradiation parameters.

11. Method according to claim 10, with the determination of the irradiation parameters p for the number of beams including a minimisation of a target function F(p)

$$F(p) = (1 - W) \cdot F_{tot}(p) + W \cdot \sum_{i} F_i(p)$$

, wherein
$F_{tot}(p)$ includes an evaluation function in respect of fulfillment of the total dose conditions for the irradiation parameters p, and
$F_i(p)$ includes an evaluation function in respect of fulfillment of the single beam dose conditions for the beam i in the irradiation parameters p.

12. Method according to one of the previous claims, further comprising a determination of a number of beam-specific irradiation areas in the object to be irradiated,
wherein the irradiation parameters for the number of beams (1403, 1404) for irradiation areas in which the beam-specific irradiation areas overlap, are determined as a function of the total dose conditions and for irradiation areas in which the beam-specific irradiation areas do not overlap, are determined as a function of the single beam dose conditions.

13. Method according to one of the preceding claims, wherein the total dose condition comprises a maximum dose, a minimum dose, a dose-volume histogram, an average dose, an equivalent uniform dose (EUD), a biological target value or a dose homogeneity.

14. Method according to one of the preceding claims, wherein the single beam dose condition comprises a maximum dose, a minimum dose or an average dose or a dose homogeneity.

15. A device for determining a radiotherapy treatment plan for an irradiation of an object to be irradiated, wherein a number of irradiation areas are defined in the object to be irradiated, wherein the object to be irradiated is irradiated with a number of beams (1403, 1404) from different directions, comprising:

- a first determination unit (907 for determining a number of total dose conditions, wherein at least one of the number of total dose conditions is assigned in each case to a respective one of the number of irradiation areas, whereby the total dose condition assigned to the respective irradiation area defines a condition for the total beam dose for the irradiation area,
- a second determination unit (908) for determining a number of single beam dose conditions, whereby at least one of the number of single beam dose conditions is assigned to a respective one of the number of beams (1403, 1404) and a respective one of the number of irradiation areas, wherein the single beam dose condition assigned to the respective beam (1403, 1404) defines a condition for the radiation dose based on the beam (1403, 1404) for the respective irradiation area, and
- a processing unit (906), which is embodied to define irradiation parameters for the number of beams (1403, 1404) as a function of the number of total dose conditions and the number of single beam dose conditions, **characterised in that** the second determination unit (980) is designed such that
the determination of the number of single beam dose conditions includes an automatic determination of a number of single beam dose conditions as a function of a predetermined beam homogeneity factor HF, wherein the beam homogeneity factor HF defines for one of the number of irradiation areas a maximum difference between radiation doses introduced by the number of beams (1403, 1404) into the irradiation area.

16. Device according to claim 15, **characterised in that** the device (903) is embodied for carrying out the method in accordance with one of claims 2-14.

17. Particle therapy system with a device (903) according to claim 15 or 16.

18. Computer program product which comprises a program and is able to be loaded directly into a memory of a programmable processing unit (906) of a device (903) for determining a radiotherapy treatment plan, with program means to execute all steps of the method according to one of claims 1-14, when the program is executed in the processing unit (906).

19. Electronically-readable data medium with electronically-readable control information stored thereon, which is embodied such that when the data medium (905) is used in a processing unit (906) of a device (903) to determine a radiotherapy treatment plan, it executes the method according to one of claims 1-14.

**Revendications**

1. Procédé de détermination d'un plan d'irradiation pour l'irradiation d'un objet à irradier dans lequel, on fixe plusieurs régions d'irradiation dans l'objet à irradier, l'objet à irradier étant irradié par plusieurs rayonnements ( 1403, 1404 ) de directions différentes,
comprenant :

   - la détermination de plusieurs conditions de dose totale, respectivement au moins l'une des plusieurs conditions de dose totale étant affectée à l'une respective des plusieurs régions d'irradiation, la condition de dose totale affectée à la région d'irradiation respective fixant une condition pour la dose d'irradiation totale de la région d'irradiation,
   - la détermination de plusieurs conditions de dose de rayonnement individuelle, respectivement au moins l'une des plusieurs conditions de dose de rayonnement individuelle étant affectée à l'une respective des plusieurs régions d'irradiation, la condition de dose de rayonnement individuelle affectée au rayonnement ( 1403, 1404 ) respectif fixant une condition pour la dose d'irradiation sur la base du rayonnement ( 1403, 1404 ) pour la région d'irradiation respective et
   - la détermination de paramètres d'irradiation pour les plusieurs rayonnements ( 1403, 1404 ) en fonction des plusieurs conditions de dose totale et des plusieurs conditions de dose de rayonnement individuelle,

   **caractérisé en ce que**

   - la détermination des plusieurs conditions de dose de rayonnement individuelle comprend une détermination automatique de plusieurs conditions de dose de rayonnement individuelle en fonction d'un facteur HF d'homogénéité du rayonnement donné à l'avance, le facteur HF d'homogénéité du rayonnement fixant pour l'une des plusieurs régions d'irradiation un écart maximum entre les doses d'irradiation apportées à la région d'irradiation par les plusieurs rayonnements ( 1403, 1404 ).

2. Procédé suivant la revendication 1, dans lequel la détermination du paramètre p d'irradiation pour les plusieurs rayonnements ( 1403, 1404 ) comprend une minimisation d'une fonction F(p) cible avec

$$F(p) = F_{tot}(p) + \sum F_i(p)$$

dans laquelle

$F_{tot}(p)$ comprend une fonction d'évaluation concernant une satisfaction des conditions de dose totale aux paramètres p d'irradiation et

$F_i(p)$ comprend une fonction d'évaluation concernant la satisfaction des conditions de dose de rayonnement individuelle pour le rayonnement i aux paramètres p d'irradiation.

3. Procédé suivant la revendication 1 ou 2, dans lequel la détermination des plusieurs conditions de dose totale comprend une détection d'une entrée ( 1104 ) d'utilisateur d'une condition de dose totale respective pour respectivement l'une des plusieurs régions d'irradiation.

4. Procédé suivant l'une des revendications précédentes, dans lequel la détermination de plusieurs conditions de dose de rayonnement individuelle comprend une détection d'une entrée ( 1104 ) d'utilisateur d'une condition de dose de rayonnement individuelle respective pour respectivement l'un des plusieurs rayonnements ( 1403, 1404 ) pour respectivement l'une des plusieurs régions d'irradiation.

5. Procédé suivant l'une des revendications précédentes, dans lequel le procédé comprend en outre une détection d'une entrée ( 1301 ) d'utilisateur, par laquelle un utilisateur prescrit le facteur HF d'homogénéité du rayonnement.

6. Procédé suivant l'une des revendications précédentes, dans lequel le facteur HF d'homogénéité du rayonnement comprend une valeur donnée à l'avance de manière fixe.

7. Procédé suivant l'une des revendications précédentes, dans lequel le procédé comprend en outre une détection d'une entrée d'utilisateur d'une pondération HW d'homogénéité du rayonnement,
la détermination des paramètres d'irradiation comportant une détermination de paramètres d'irradiation pour chacun des plusieurs rayonnements ( 1403, 1404 ) en fonction des plusieurs conditions de dose totale, des plusieurs conditions de dose de rayonnement individuelle et de la pondération HW d'homogénéité du rayonnement.
la pondération HW d'homogénéité du rayonnement fixant une valeur pour la prise en compte des conditions de dose de rayonnement individuelle lors de la détermination des paramètres d'irradiation.

8. Procédé suivant la revendication 7, dans lequel la détermination des paramètres p d'irradiation pour les plusieurs rayonnements comprend une minimisation d'une fonction F(p) cible avec

$$F(p) = F_{tot} + HW \sum_i F_i(p, HF)$$

dans laquelle

$F_{tot}(p)$ comprend une fonction d'évaluation concernant la satisfaction des conditions de dose totale aux paramètres p d'irradiation et

$F_i(p, HF)$ comprend une fonction d'évaluation définie par le facteur HF d'homogénéité du rayonnement concernant la satisfaction des conditions de dose de rayonnement individuelle pour le rayonnement i aux paramètres p d'irradiation.

9. Procédé suivant la revendication 8, dans lequel on définit la fonction $F_i(p, HF)$ d'évaluation définie par le facteur HF d'homogénéité du rayonnement pour le rayonnement i sur la base d'un niveau $D_{min,tot}$ de dose totale minimale donnée à l'avance et d'un niveau $D_{max,tot}$ de dose totale maximale donnée à l'avance, la fonction d'évaluation de dose pour le rayonnement i étant définie par un niveau $D_{max,i}$ de dose pour le faisceau i et par un niveau $D_{min,i}$ de dose minimum pour le faisceau i suivant les équations :

$$D_{max} = D_{max,tot} * (1 - HF/B)$$

et

$$D_{min,I} = D_{min,tot} *HF/B$$

B étant le nombre des rayonnements ( 1403, 1404 ).

10. Procédé suivant l'une des revendications précédentes, dans lequel le procédé comprend en outre une détection d'une entrée ( 1302 ) d'utilisateur d'une pondération W du rayonnement individuelle, dans lequel la détermination des paramètres d'irradiation comprend une détermination de paramètres d'irradiation pour chacun des plusieurs rayonnements ( 1403, 1404 ) en fonction des plusieurs conditions de dose totale, des plusieurs conditions de dose de rayonnement individuelle et de la pondération du rayonnement individuelle, dans lequel la pondération W du rayonnement individuelle fixe un rapport entre une prise en compte des conditions de dose de rayonnement individuelle et une prise en compte des conditions de dose totale lors de la détermination des paramètres d'irradiation.

11. Procédé suivant la revendication 10, dans lequel la détermination des paramètres p d'irradiation pour les plusieurs rayonnements comprend une minimisation d'une fonction F(p) cible avec

$$F(p) = (1-W) F_{tot}(p) + W \sum_i F_i(p)$$

dans laquelle
$F_{tot}(p)$ comprend une fonction d'évaluation concernant la satisfaction des conditions de dose totale aux paramètres p d'irradiation et
$F_i(p)$ comprend une fonction d'évaluation concernant la satisfaction des conditions de dose de rayonnement individuelle pour le rayonnement i aux paramètres p d'irradiation.

12. Procédé suivant l'une des revendications précédentes, comprenant en outre une détermination de plusieurs régions d'irradiation spécifiques au rayonnement dans l'objet à irradier, dans lequel on détermine les paramètres d'irradiation pour les plusieurs rayonnements ( 1403, 1404 ) pour des régions d'irradiation, dans lesquelles les régions d'irradiation spécifiques au rayonnement se chevauchent, en fonction des conditions de dose totale et, pour les régions d'irradiation dans lesquelles les régions d'irradiation spécifiques aux rayonnements ne se chevauchent pas, en fonction des conditions de dose de rayonnement individuelle.

13. Procédé suivant l'une des revendications précédentes, dans lequel la condition de dose totale comprend une dose maximum et une dose minimum, un histogramme dose-volume, une dose moyenne, une dose uniforme équivalente ( EUD ), une grandeur cible biologique ou une homogénéité de dose.

14. Procédé suivant l'une des revendications précédentes, dans lequel la condition de dose de rayonnement individuelle comprend une dose maximum, une dose minimum ou une dose moyenne ou une homogénéité de dose.

15. Dispositif de détermination d'un plan d'irradiation pour l'irradiation d'un objet à irradier, dans lequel plusieurs régions d'irradiation sont fixées dans l'objet à irradier, l'objet à irradier étant irradié par plusieurs rayonnements ( 1403, 1404 ) de directions différentes, comprenant :

- une première unité ( 907 ) de détermination de plusieurs conditions de dose totale respectivement au moins l'une des plusieurs conditions de dose totale étant affectée à l'une respective des plusieurs régions d'irradiation, la condition de dose totale affectée à la région d'irradiation respective fixant une condition pour la dose de rayonnement total pour la région d'irradiation,
- une deuxième unité ( 908 ) de détermination de plusieurs conditions de dose de rayonnement individuelle respectivement au moins l'une des plusieurs conditions de dose de rayonnement individuelle étant affectée à l'un respectif de plusieurs rayonnements ( 1403, 1404 ) et à l'une respective des plusieurs régions d'irradiation, la condition de dose de rayonnement individuelle affectée aux rayonnements ( 1403, 1404 ) respectifs fixant une condition pour la dose de rayonnement sur la base du rayonnement ( 1403, 1404 ) pour la région d'irradiation respective et

- une unité ( 906 ) de traitement, qui est conformée pour déterminer des paramètres d'irradiation pour les plusieurs rayonnements ( 1403, 1404 ) en fonction des plusieurs conditions de dose totale et des plusieurs conditions de dose de rayonnement individuelle,

**caractérisé en ce que** la deuxième unité ( 908 ) de détermination est conçue de manière à ce que la détermination des plusieurs conditions de dose de rayonnement individuelle comprenne une détermination automatique de plusieurs conditions de dose de rayonnement individuelle en fonction d'un facteur HF d'homogénéité du rayonnement donné à l'avance, le facteur HF d'homogénéité du rayonnement fixant pour l'une des plusieurs régions d'irradiation un écart maximum entre des doses de rayonnements apportées à la région d'irradiation par les plusieurs rayonnements ( 1403, 1404 ).

16. Dispositif suivant la revendication 15, **caractérisé en ce que** le dispositif ( 903 ) est conformé pour effectuer les procédés suivant l'une des revendications 2 à 14.

17. Installation d'irradiation par des particules, comprenant un dispositif ( 903 ) suivant la revendication 15 ou 16.

18. Produit de programme d'ordinateur, qui comprend un programme et qui peut être chargé directement dans une mémoire d'une unité ( 906 ) de traitement programmable d'un dispositif ( 903 ) de détermination d'un plan d'irradiation comprenant des moyens de programme pour effectuer tous les stades du procédé suivant l'une des revendications 1 à 14, lorsque le programme est réalisé dans l'unité ( 906 ) de traitement.

19. Support de données déchiffrables électroniquement, sur lequel sont mémorisées des informations de commande pouvant être déchiffrées électroniquement, lesquelles sont conformées de manière à ce que, lorsque l'on utilise le support ( 905 ) de données dans une unité ( 906 ) de traitement d'un dispositif ( 903 ) de détermination d'un plan d'irradiation, elles effectuent le procédé suivant l'une des revendications 1 à 14.

FIG 1

FIG 2

FIG 3

FIG 4

FIG 5

FIG 6

FIG 7

FIG 8

# FIG 9

Strahlsteuerung

901

906   904

Verarbeitungseinheit

907   908

903   902

905

# FIG 10

| Gesamtdosisbedingungen bestimmen | ~1001 |
| Einzelstrahldosisbedingungen bestimmen | ~1002 |
| Bestrahlungsparameter bestimmen | ~1003 |

# FIG 11

903

1101

1103

|    | Min | Max |
|----|-----|-----|
| T  | 3   | 4   |
| 0₁ | ... | ... |
| ...| ... | ... |

~1104

1105

# FIG 12

|   | Min | Max |
|---|-----|-----|
| T | 3 | 4 |
| 0 1 | ... | ... |
| ... | ... | ... |

# FIG 13

**Strahlenhomogenität**

gering                                                    hoch

1301

**Einzelstrahlgewichtung**

hoch                                                    gering

SBO                                                    IMPT

1302

FIG 14

FIG 15

FIG 16

FIG 17

FIG 18

FIG 19

FIG 20

FIG 21

FIG 22

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7268358 B2

**In der Beschreibung aufgeführte Nicht-Patentliteratur**

- **MARTIN SOUKUP et al.** Study of Robustness of IMPT and IMRT for Prostate Cancer against Organ Movement. *Int J Radiat Oncol Biol Phys.,* 2009, vol. 75 (3), 941-9

- **F. ALBERTINI et al.** Degeneracy and Robustness of IMPT Plans in the Treatment of Skull-Base Chordomas. *Med. Phys.,* 2007, vol. 34 (6), 2431-2431